# EUROPEAN PATENT APPLICATION

(11) **EP 1 695 706 A1**
(43) Date of publication of application: **30.08.2006**
(21) Application number: 04807516.2
(22) Date of filing: 15.12.2004
(51) Int. Cl.: A61K 31/4375, A61K 31/366, A61K 31/40, A61K 31/404, A61K 31/47, A61K 31/505, A61K 31/397, A61K 45/00, A61K 31/22, A61P 3/06, A61P 9/10, A61P 43/00

(54) **MEDICINAL COMPOSITIONS AND COMBINATIONS**

(30) Priority: 17.12.2003 JP 2003420083
(71) Applicant: Dainippon Sumitomo Pharma Co., Ltd., Osaka 541-8524 (JP)
(72) Inventor: KINO,Kouichi Dainippon Sumitomo Pharma Co.,Ltd, Osaka-shi Osaka 5540022 (JP); IORIYA,Katsuhisa Dainippon Sumitomo Pharma Co.,Ltd, Osaka-shi Osaka 5540022 (JP)
(74) Representative: Vossius & Partner
(86) International application number: PCT/JP2004/019159
(87) International publication number: WO 2005/058316

(57) **Abstract**

A therapy of hyperlipemia or arteriosclerosis by using a pharmaceutical composition comprising
(A) 3-hydroxy-3-methylglutaryl coenzyme A reductase inhibitor or a salt thereof etc., and/or ezetimibe or a prodrug thereof or a salt thereof; and
(B) a compound of the formula (I): 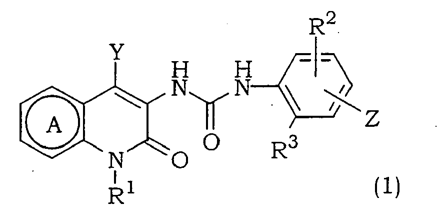
wherein Ring A is a substituted or unsubstituted pyridine ring etc., Y is a substituted or unsubstituted aryl etc., R¹ is a hydrogen atom, or a substituted or unsubstituted alkyl etc., R² is a hydrogen atom or a lower alkyl group, R³ is a lower alkyl group, and Z is a hydroxy group or a amino group etc., or a salt thereof etc.; or
a combination of (A) and (B),
which is effective for a therapy of a patient who are not remedied by the current therapeutic agent, i. e. being not decreased to the desired level, in particular a patient having already suffered from a coronary heart disease etc.

## Description

### TECHNICAL FIELD

This invention relates to an agent for hyperlipidemia and arteriosclerosis.

### PRIOR ART

Coronary heart disease is a major cause of death in advanced countries. Hypercholesterolemia has been confirmed as the major risk factor of this coronary heart disease mainly by many epidemiologic studies and also the increase of the amounts of plasma total cholesterol and plasma low-density lipoprotein (LDL) cholesterol are linked closely to a coronary heart disease according to the results of extensive prevention trials reported recently (Arterioscler. Thromb. Vasc. Biol. (1999) vol. 19, 187-195). Thus the lowering of cholesterol in plasma reduces the arteriosclerotic disease and results in reducing the risk of onset of a coronary heart disease and then many antihypercholesterolemic agents have clinically been used. In such a situation, the targeted level for therapy of LDL cholesterol is defined on the basis of the number of risk factor included in an individual patient according to the National Cholesterol Education Program (NECP) in U. S.. However, said targeted level for therapy is not achieved well by the current therapeutic agent, and in particular, the degree of achievement of the targeted level is very low in the patients with a history of coronary heart disease (Arch. Intern. Med. (2000) vol.160, 459-467). It is well known that LDL receptor is a receptor essential for uptake of LDL from the blood into cell and the activity of LDL receptor in liver play a major role in controlling a cholesterol level in blood. Thus, the agent exhibiting an action for elevating the expression of LDL receptor can lower the cholesterol in plasma. The most well known agent having such activity is 3-hydroxy-3-methylglutaryl coenzyme A reductase inhibitor (hereinafter, occasionally, referred to as HMG-CoA reductase inhibitor).

HMG-CoA reductase inhibitor inhibits the biosynthesis of cholesterol in cells and thereby it activates indirectly the transcription of LDL receptor gene and then promotes the expression of the LDL receptor (Science (1986) vol. 232, 34-47). Although it's usefulness as antihypercholesterolemic agent has been demonstrated in many clinical trials on large scale, the activity thereof is limited even if the dosage increases, but on the other hand, the increase of dosage causes occurrence of the side effects such as a muscle disorder and a rhabdomyolysis, and therefore there is a limit of the reduction of LDL cholesterol by a single agent. Thus, it has been desired to develop a method for elevating an expression of LDL receptor in more safety and higher degree.

By the way, Ezetimibe (Chemical name: 1-(4-fluorophenyl)-3(R)-[3-(4-fluorophenyl)-3(S)-hydroxypropyl]-4(S)-(4-hydroxyphenyl)-2-azetidinone) is commercially available as a cholesterol lowering agent by the Merck/ Schering-Plough Pharmaceuticals in the United States of America. Ezetimibe exhibits the lipid lowering activity due to the inhibition of absorption of cholesterol from the intestinal tract. Besides, Niemann-Pick C1 Like 1 protein (Science, 2004, vol. 303, p.1201-1204) and Aminopeptidase N (Journal of Biological Chemistry, 2004, in press) were reported as the target molecule. However, it is reported in European Atherosclerosis Society Congress, 2002 that the degree of lowering of LDL cholesterol by a single agent of ezetimibe was merely in 15 to 20% and it could not reduce more even if the dosage was increased. Then it will be assumed that the targeted level of therapy cannot be often achieved by a single agent of ezetimibe. Thus, it has been desired to develop a method for elevating safely the expression of LDL receptor and lowering more the cholesterol.

On the other hand, it is known that the compound of the formula (1) or the prodrug thereof or a salt of the same (hereinafter, occasionally, referred to as "compound of the formula (1) etc.") exhibit an activity inhibiting acyl-CoA: cholesterol acyltransferase (ACAT) and is also useful as an agent for hyperlipidemia and arteriosclerosis etc. (for example, PCT International Publication No. 00/09505 pamphlet). However, in the said pamphlet, it is not found any specific description with respect to composition or combination with an HMG-CoA reductase inhibitor or ezetimibe.

### DISCLOSURE OF INVENTION

An object of the present invention is to provide a pharmaceutical composition making it possible to achieve the desired targeted level of therapy which could not be achieved by the above current therapeutic agents. We have unexpectedly found that a combination of (A) HMG-CoA reductase inhibitor and a prodrug thereof, or a salt of the same (hereinafter, occasionally, referred to as "HMG-CoA reductase inhibitor etc.") and/or ezetimibe or a prodrug thereof, or a salt of the same (hereinafter, occasionally, referred to as "ezetimibe etc.") and (B) the compound of the formula (1) etc. make it possible to promote the expression of the LDL receptor which cannot be achieved by using each single drug, and thus the present invention has been completed. That is, the present invention relates to the following embodiments:
[1] An agent for treating hyperlipidemia or arteriosclerosis comprising
   (A) 3-hydroxy-3-methylglutaryl coenzyme A reductase inhibitor or a prodrug thereof or a pharmaceutically acceptable salt of the same and/or ezetimibe or a prodrug thereof or a pharmaceutically acceptable salt of the same; and
   (B) a compound of the formula (1): 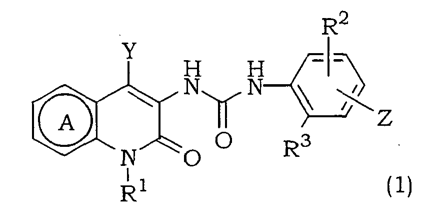
   wherein Ring A is a substituted or unsubstituted pyridine ring;
   Y is a substituted or unsubstituted alkyl group, a substituted or unsubstituted cycloalkyl group, or a substituted or unsubstituted aromatic group;
   R¹ is a hydrogen atom, a substituted or unsubstituted alkyl group, a substituted or unsubstituted alkenyl group, a substituted or unsubstituted alkynyl group, or a substituted or unsubstituted cycloalkyl group;
   R² is a hydrogen atom or a lower alkyl group;
   R³ is a lower alkyl group;
   Z is a group represented by either of the following formula 1) or 2):

      1) ―D¹―Q

      wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸― (R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or

      2) ―D²―M―E―W

      wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶― (R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond, or a prodrug thereof, or a pharmaceutically acceptable salt of the same.
[2] The agent for hyperlipidemia or arteriosclerosis described in [1], wherein in the formula (1), Ring A is one of the groups of the following formulae (a), (b) and (c): 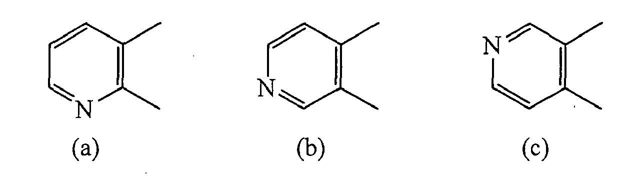
   Y is a substituted or unsubstituted aromatic group;
   R¹ is a substituted or unsubstituted alkyl group, or a substituted or unsubstituted alkenyl group;
   Z is a group of the formula: ―D¹―Q, wherein the D¹ is a direct bond, Q is a hydroxy group or a group of the formula: ―NR⁴R⁵.
[3] The agent for hyperlipidemia or arteriosclerosis described in [1] or [2], wherein the compound of formula (1) is represented by the formula (51): 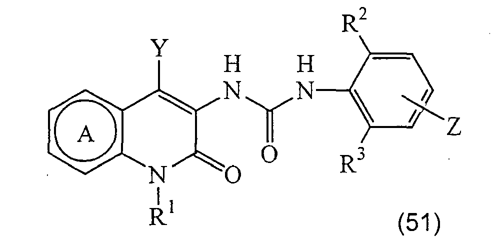 wherein the Ring A, R¹, R², R³ and Z have the same meanings as defined in [1]; Y is a phenyl group substituted by a group represented by the formula ―M¹―E¹―T, wherein M¹ is an oxygen atom, E¹ is a hydrocarbon group having 2 to 4 carbon atoms, T is a hydroxy group or a group represented by the formula ―NR⁴¹R⁵¹ (R⁴¹ and R⁵¹ are independently a hydrogen atom, a lower alkoxy-substituted or unsubstituted lower alkyl group, a cycloalkyl group, a lower alkoxycarbonyl group, or an aralkyl group, or alternatively R⁴¹ and R⁵¹ may combine each other, and with the adjacent nitrogen atom to which they bond, form a saturated cyclic amino group having 4 to 8 carbon atoms as ones forming the said ring, and optionally having one ―NR⁸¹― (R⁸¹ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof.
[4] The agent for hyperlipidemia or arteriosclerosis described in [1], wherein the compound of formula (1) is N-[1-butyl-4-[3-[3-(hydroxy)propoxy]phenyl]-1,2-dihydro-2-oxo-1,8-naphthyridin-3-yl]-N'-(2,6-diisopropyl-4-aminophenyl)urea.
[5] The agent for hyperlipidemia or arteriosclerosis described in any one of [1] to [4], wherein 3-hydroxy-3-methylglutaryl coenzyme A reductase inhibitor is selected from the group consisting of pravastatin, simvastatin, lovastatin, fluvastatin, atorvastatin, rosuvastatin, and pitavastatin.
[6] An agent for hyperlipidemia or arteriosclerosis comprising a compound of the formula (1): 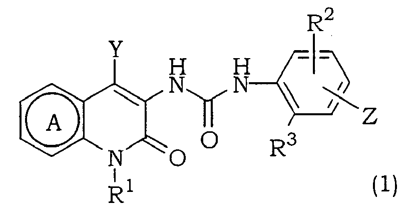 wherein Ring A is a substituted or unsubstituted pyridine ring;
   Y is a substituted or unsubstituted alkyl group, a substituted or unsubstituted cycloalkyl group, or a substituted or unsubstituted aromatic group;
   R¹ is a hydrogen atom, a substituted or unsubstituted alkyl group, a substituted or unsubstituted alkenyl group, a substituted or unsubstituted alkynyl group, or a substituted or unsubstituted cycloalkyl group;
   R² is a hydrogen atom or a lower alkyl group;
   R³ is a lower alkyl group;
   Z is a group represented by either of the following formula 1) or 2):

      1) ―D¹―Q

      wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸― (R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or

      2) ―D²―M―E―W

      wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶―(R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond,
   or a prodrug thereof or a pharmaceutically acceptable salt of the same, to be used in combination with a pharmaceutical composition comprising 3-hydroxy-3-methylglutaryl coenzyme A reductase inhibitor or a prodrug thereof or a pharmaceutically acceptable salt of the same and/or ezetimibe or a prodrug thereof or a pharmaceutically acceptable salt of the same.
[7] A pharmaceutical composition for potentiating a blood cholesterol lowering action to be used in a therapy using a pharmaceutical composition comprising 3-hydroxy-3-methylglutaryl coenzyme A reductase inhibitor or a prodrug thereof or a pharmaceutically acceptable salt of the same, and/or ezetimibe or a prodrug thereof or a pharmaceutically acceptable salt of the same, which comprises a compound of the formula (1): 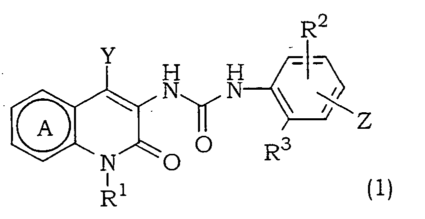 wherein Ring A is a substituted or unsubstituted pyridine ring;
   Y is a substituted or unsubstituted alkyl group, a substituted or unsubstituted cycloalkyl group, or a substituted or unsubstituted aromatic group;
   R¹ is a hydrogen atom, a substituted or unsubstituted alkyl group, a substituted or unsubstituted alkenyl group, a substituted or unsubstituted alkynyl group, or a substituted or unsubstituted cycloalkyl group;
   R² is a hydrogen atom or a lower alkyl group;
   R³ is a lower alkyl group;
   Z is a group represented by either of the following formula 1) or 2):

      1) ―D¹―Q

      wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸―(R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or

      2) ―D²―M―E―W

      wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶―(R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond,
   or a prodrug thereof or a pharmaceutically acceptable salt of the same.
[8] An agent for treating hyperlipidemia or arteriosclerosis comprising 3-hydroxy-3-methylglutaryl coenzyme A reductase inhibitor or a prodrug thereof or a pharmaceutically acceptable salt of the same, and/or ezetimibe or a prodrug thereof or a pharmaceutically acceptable salt of the same, which is used in combination with a pharmaceutical composition comprising a compound of the formula (1): 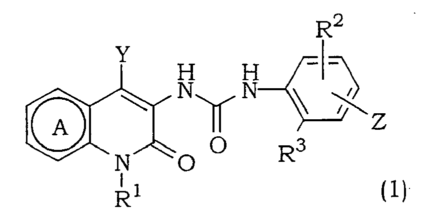 wherein Ring A is a substituted or unsubstituted pyridine ring;
   Y is a substituted or unsubstituted alkyl group, a substituted or unsubstituted cycloalkyl group, or a substituted or unsubstituted aromatic group;
   R¹ is a hydrogen atom, a substituted or unsubstituted alkyl group, a substituted or unsubstituted alkenyl group, a substituted or unsubstituted alkynyl group, or a substituted or unsubstituted cycloalkyl group;
   R² is a hydrogen atom or a lower alkyl group;
   R³ is a lower alkyl group;
   Z is a group represented by either of the following formula 1) or 2):

      1) ―D¹―Q

      wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸―(R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or

      2) ―D²―M―E―W

      wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶―(R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond,
   or a prodrug thereof or a pharmaceutically acceptable salt of the same.
[9] A pharmaceutical composition for potentiating a blood cholesterol lowering action comprising 3-hydroxy-3-methylglutaryl coenzyme A reductase inhibitor or a prodrug thereof or a pharmaceutically acceptable salt of the same, and/or ezetimibe or a prodrug thereof or a pharmaceutically acceptable salt of the same, which is used in a therapy using a pharmaceutical composition comprising a compound of the formula (1): 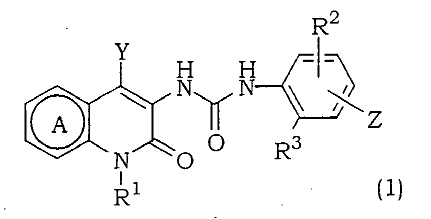 wherein Ring A is a substituted or unsubstituted pyridine ring;
   Y is a substituted or unsubstituted alkyl group, a substituted or unsubstituted cycloalkyl group, or a substituted or unsubstituted aromatic group;
   R¹ is a hydrogen atom, a substituted or unsubstituted alkyl group, a substituted or unsubstituted alkenyl group, a substituted or unsubstituted alkynyl group, or a substituted or unsubstituted cycloalkyl group;
   R² is a hydrogen atom or a lower alkyl group;
   R³ is a lower alkyl group;
   Z is a group represented by either of the following formula 1) or 2):

      1) ―D¹―Q

      wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸― (R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or

      2) ―D²―M―E―W

      wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶―(R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond, or a prodrug thereof or a pharmaceutical acceptable salt of the same.
[10] A commercial package which comprises a pharmaceutical composition comprising a compound of the formula (1): 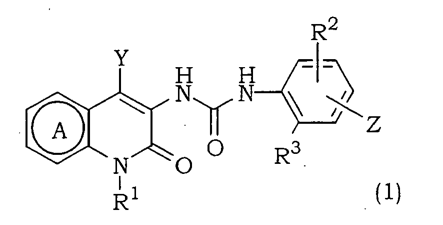 wherein Ring A is a substituted or unsubstituted pyridine ring;
   Y is a substituted or unsubstituted alkyl group, a substituted or unsubstituted cycloalkyl group, or a substituted or unsubstituted aromatic group;
   R¹ is a hydrogen atom, a substituted or unsubstituted alkyl group, a substituted or unsubstituted alkenyl group, a substituted or unsubstituted alkynyl group, or a substituted or unsubstituted cycloalkyl group;
   R² is a hydrogen atom or a lower alkyl group;
   R³ is a lower alkyl group;
   Z is a group represented by either of the following formula 1) or 2):

      1) ―D¹―Q

      wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸― (R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or

      2) D²―M―E―W

      wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶―(R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond, or a prodrug thereof or a pharmaceutically acceptable salt of the same, and a package insert indicating that said pharmaceutical composition may be used or should be used for potentiating a blood cholesterol lowering action with 3-hydroxy-3-methylglutaryl coenzyme A reductase inhibitor or a prodrug thereof or a pharmaceutically acceptable salt of the same, and/or ezetimibe or a prodrug thereof or a pharmaceutically acceptable salt of the same.
[11] A commercial package which comprises a pharmaceutical composition comprising 3-hydroxy-3-methylglutaryl coenzyme A reductase inhibitor or a prodrug thereof or a pharmaceutically acceptable salt of the same, and/or ezetimibe or a prodrug thereof or a pharmaceutically acceptable salt of the same,
   and a package insert indicating that said pharmaceutical composition may be used or should be used for potentiating a blood cholesterol lowering action with a compound of the formula (1): 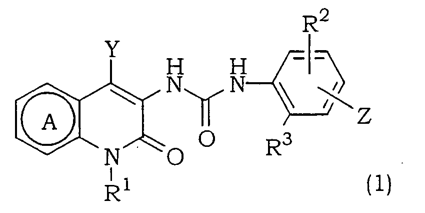 wherein Ring A is a substituted or unsubstituted pyridine ring;
   Y is a substituted or unsubstituted alkyl group, a substituted or unsubstituted cycloalkyl group, or a substituted or unsubstituted aromatic group;
   R¹ is a hydrogen atom, a substituted or unsubstituted alkyl group, a substituted or unsubstituted alkenyl group, a substituted or unsubstituted alkynyl group, or a substituted or unsubstituted cycloalkyl group;
   R² is a hydrogen atom or a lower alkyl group;
   R³ is a lower alkyl group;
   Z is a group represented by either of the following formula 1) or 2):

      1) ―D¹―Q

      wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸― (R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or

      2) ―D²―M―E―W

      wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶―(R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond, or a prodrug thereof or a pharmaceutically acceptable salt of the same.
[12] A commercial package which comprises a combination of
   (A) a pharmaceutical composition comprising a compound of the formula (1): 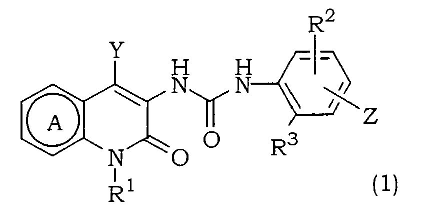 wherein Ring A is a substituted or unsubstituted pyridine ring;
      Y is a substituted or unsubstituted alkyl group, a substituted or unsubstituted cycloalkyl group, or a substituted or unsubstituted aromatic group;
      R¹ is a hydrogen atom, a substituted or unsubstituted alkyl group, a substituted or unsubstituted alkenyl group, a substituted or unsubstituted alkynyl group, or a substituted or unsubstituted cycloalkyl group;
      R² is a hydrogen atom or a lower alkyl group;
      R³ is a lower alkyl group;
      Z is a group represented by either of the following formula 1) or 2):

         1) ―D¹―Q

         wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸―(R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or

         2) ―D²―M―E―W

         wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶―(R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond,
      or a prodrug thereof or a pharmaceutically acceptable salt of the same; and
   (B) 3-hydroxy-3-methylglutaryl coenzyme A reductase inhibitor or a prodrug thereof or a pharmaceutically acceptable salt of the same, and/or ezetimibe or a prodrug thereof or a pharmaceutically acceptable salt of the same;
      and a package insert indicating that said combination may be used or should be used for lowering blood cholesterol.

This invention provide a method for increasing the expression of LDL receptor more safely, and more effectively in comparison with the case of using a single agent composed of each of HMG-CoA reductase inhibitor etc., ezetimibe etc., or compound of formula (1) etc.. Concurrently, this invention provides an agent for treating hyperlipidemia or arteriosclerosis comprising (A) HMG-CoA reductase inhibitor etc., and/or ezetimibe etc., and (B) a compound of formula (1) etc.; an agent for treating hyperlipidemia or arteriosclerosis which comprises either one of (A) or (B) which is used in combination with another one; and a pharmaceutical composition for potentiating the blood cholesterol lowering action comprising either one of (A) or (B), which is used in a therapy with a pharmaceutical composition comprising another one.

As mentioned above, in the case of using HMG-CoA reductase inhibitor alone, the dosage is limited in view of the side effect. Actually, as is shown in the following Examples, when, either one of HMG-CoA reductase inhibitor etc. and the compound of the formula (1) etc. was used alone, it could not give enhancement of the effect even if the dosage was increased, but when these were used in a combination thereof, they exhibited the remarkable increase of the actions of binding, uptake and degradation of LDL, the lowering of cholesterol in plasma as well as the increase of the expression of LDL receptor protein.

These effects have first been found by the present inventors who have had the concept of use of combination of the HMG-CoA reductase inhibitor etc. and the compound of the formula (1) etc. and have studied about the combination, which would have never been predicted by any person skilled in the art.

### BRIEF DECRIPTION OF THE DRAWINGS

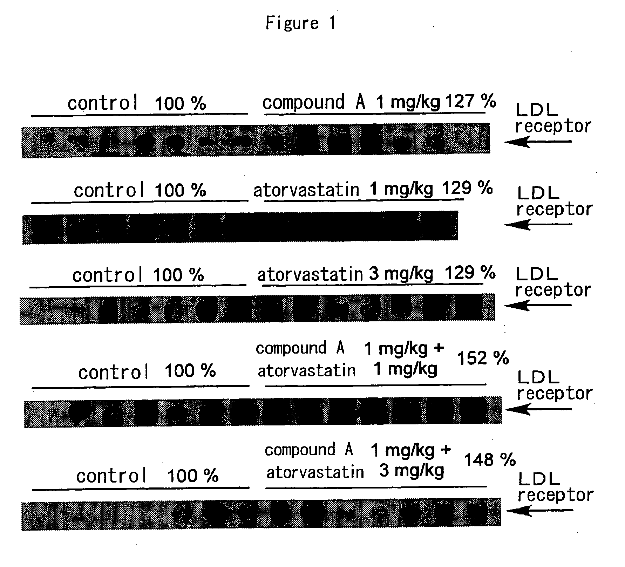

Figure 1 shows a result of an immunoblotting in Example 3, which was obtained by reading the X ray film by a scanner, said film being visualized by the chemiluminescence technique. The values in the figure represents as a relative mean value, wherein said mean value was obtained by quantitating the shading of the band by the image analysis and shown in relation to the mean value in control which is counted as 100%.

### BEST MODE FOR CARRING OUT AN INVENTION

Each term in the present invention is explained below. Unless defined otherwise, the definition for each group should be applied to cases wherein said group is a part of another substituent.

HMG-CoA reductase inhibitor includes pravastatin, simvastatin, lovastatin, fluvastatin, atorvastatin, rosuvastatin, and pitavastatin.

The term "a blood cholesterol lowering action" in the present invention includes for example, a lowering action of total cholesterol in blood and a lowering action of low-density lipoprotein (LDL) cholesterol in blood, and the like.

The term "a therapy for hyperlipemia" in the present invention includes for example, a therapy for hypercholesterolemia.

The term "lower" in the present invention means that an alkyl moiety described with "lower" is a lower alkyl group, and the lower alkyl group includes a lower alkyl group having 1 to 6 carbon atoms such as methyl, ethyl, propyl, 2-propyl, butyl, t-butyl, pentyl, hexyl, etc.

The halogen atom includes fluorine atom, chlorine atom, bromine atom, or iodine atom.

Ring A is a substituted or unsubstituted pyridine ring, and the nitrogen atom thereof may be at any position except for the fused positions of the fused ring, and the preferable Ring A is one of the groups of the following formulae (a), (b) and (c):

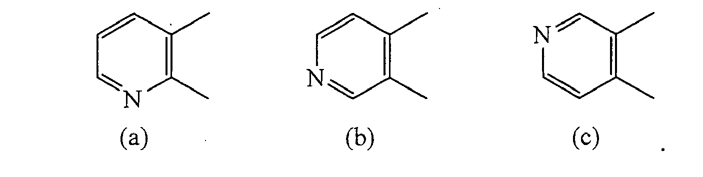

The substituent of the pyridine ring may be, for example, a lower alkyl group, a halogen atom, a cyano group, a trifluoromethyl group, a nitro group, an amino group, a mono-lower alkylamino group, a di-lower alkylamino group, a hydroxy group, a lower alkoxy group, a lower alkylthio group, a lower alkylsulfinyl group, a lower alkylsulfonyl group, etc. The substituted pyridine ring has one or more substituents which are the same or different.

The alkyl group includes, for example, a straight chain or branched chain alkyl group having 1 to 15 carbon atoms, such as methyl, ethyl, propyl, isopropyl, butyl, 2-butyl, 2-methylpropyl, 1,1-dimethylethyl, pentyl, 3-pentyl, 3-methylbutyl, hexyl, 3-hexyl, 4-methylpentyl, 4-heptyl, octyl, 4-octyl, decyl, undecyl, pentadecyl, etc.

The alkenyl group includes, for example, a straight chain or branched chain alkenyl group having 2 to 15 carbon atoms, such as vinyl, allyl, 2-propenyl, 2-methyl-2-propenyl, 2-butenyl, 3-butenyl, 3-methyl-2-butenyl, 4-pentenyl, 3-hexenyl, 3-ethyl-2-pentenyl, 4-ethyl-3-hexenyl, etc.

The alkynyl group includes, for example, a straight chain or branched chain alkynyl group having 3 to 15 carbon atoms, such as 2-propynyl, 3-butynyl, 4-pentynyl, 3-hexynyl, 5-methyl-2-hexynyl, 6-methyl-4-heptynyl, etc.

The cycloalkyl group includes, for example, a cycloalkyl group having 3 to 8 carbon atoms, such as cyclopropyl, cyclobutyl, cyclopentyl, cyclohexyl, cycloheptyl, cyclooctyl, etc.

The aromatic group for Y includes, for example, an aryl group and a heteroaryl group.

The aryl group includes, for example, an aryl group having carbon atoms of not more than 10, such as phenyl group, naphthyl group, etc.

The heteroaryl group includes, for example, a 5- to 6-membered monocyclic group having 1 to 2 nitrogen atoms, a 5- to 6-membered monocyclic group having 1 to 2 nitrogen atoms and one oxygen atom or one sulfur atom, a 5-membered monocyclic group having one oxygen atom or one sulfur atom, a bicyclic group formed by fusing a 6-membered ring and a 5- or 6-membered ring and having 1 to 4 nitrogen atoms, such as 2-pyridyl, 3-pyridyl, 4-pyridyl, 2-imidazolyl, pyrazinyl, 2-pyrimidinyl, 3-pyridazinyl, 3-oxadiazolyl, 2-thiazolyl, 3-isothiazolyl, 2-oxazolyl, 3-isoxazolyl, 2-furyl, 3-furyl, 2-thienyl, 3-thienyl, 2-quinolyl, 8-quinolyl, 2-quinazolinyl, 8-purinyl, etc.

The substituted aromatic group has one or more substituents which are the same or different, and the substituents are, for example, a halogen atom, a cyano group, a trifluoromethyl group, a nitro group, a hydroxy group, a methylenedioxy group, a lower alkyl group, a lower alkoxy group, a benzyloxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a carboxyl group, a lower alkoxycarbonyl group, a lower alkylthio group, a lower alkylsulfinyl group, a lower alkylsulfonyl group, a lower alkanoylamino group, a lower alkylsulfonamido group, or a group of the formula: ―M¹―E¹―T {M¹ is a direct bond, an oxygen atom, a sulfur atom, or a group of the formula: ―NR⁶¹―(R⁶¹ is a hydrogen atom or a lower alkyl group), E¹ is a divalent hydrocarbon group having 1 to 8 carbon atoms and optionally containing an unsaturated bond, T is a hydroxy group, a halogen atom, a carboxyl group, a lower alkoxycarbonyl group, a benzyloxycarbonyl group, a cyano group, a benzyloxy group, a lower alkoxy group, a lower alkanoyloxy group, a lower alkylthio group, a lower alkylsulfinyl group, a lower alkylsulfonyl group, a methanesulfonyloxy group, an alkylsubstituted or unsubstituted benzenesulfonyloxy group, a lower alkanoylamino group, a lower alkoxycarbonylamino group, a lower alkylsulfonamido group, a phthalimido group, a substituted or unsubstituted heteroaryl group, or a group of the formula: ―NR⁴¹R⁵¹ (R⁴¹ and R⁵¹ are independently a hydrogen atom, a lower alkoxy-substituted or unsubstituted lower alkyl group, a cycloalkyl group, a lower alkoxycarbonyl group, or an aralkyl group, or R⁴¹ and R⁵¹ may combine each other, and with the adjacent nitrogen atom to which they bond, form a saturated cyclic amino group having 4 to 8 carbon atoms as ones forming the said ring, and optionally having one ―NR⁸¹― (R⁸¹ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), or a group of the formula: ―C(=O)NR⁴¹R⁵¹ (R⁴¹ and R⁵¹ are as defined above)}.

The substituted lower alkyl group, the substituted phenyl group and the substituted benzyl group for R⁸ or R⁸¹ have one or more substituents which are the same or different, and the substituents includes, for example, a hydroxy group, a halogen atom, or a lower alkoxy group.

The divalent hydrocarbon group having 1 to 8 carbon atoms and optionally containing an unsaturated bond includes, for example, an alkylene chain such as methylene, ethylene, trimethylene, tetramethylene, pentamethylene, hexamethylene, etc., an alkenylene chain such as propenylene, butenylene, etc., or an alkynylene chain such as ethynylene, propynylene, butynylene, etc.

The divalent hydrocarbon group having 2 to 4 carbon atoms includes, for example, an alkylene chain such as ethylene, trimethylene, tetramethylene, an alkenylene chain such as propenylene, butenylene, etc., or an alkynylene chain such as ethynylene, propynylene, butynylene, etc.

The heteroaryl group for Q, W or T includes, for example, a 5- to 6-membered cyclic group having 1 to 3 nitrogen atoms, a 5-membered cyclic group having one oxygen atom or one sulfur atom, or a bicyclic group formed by fusing a 6-membered ring and a 5- or 6-membered ring, and having 1 to 4 nitrogen atoms, such as 1-pyrrolyl, 1-pyrazolyl, 1-imidazolyl, 1,2,4-triazol-1-yl, 2-pyridyl, 3-pyridyl, 4-pyridyl, 2-thienyl, 3-thienyl, 2-furyl, 3-furyl, 2-quinolyl, etc. The substituted heteroaryl group for Q, W or T has one or more substituents which are the same or different, and the substituents includes, for example, a lower alkyl group, a lower alkoxy group, or a halogen atom.

The cyclic amino group formed by ―NR⁴R⁵ or ―NR⁴¹R⁵¹ includes, for example, a group having 6 atoms as ones forming a ring, i.e., a 6-membered cyclic group such as 1-piperidinyl, 4-morpholinyl, 4-lower alkyl-1-piperazinyl, 4-phenyl-1-piperazinyl, or 4-benzyl-1-piperazinyl, etc., a 5-membered cyclic group such as 1-pyrrolidinyl, or a 7-membered cyclic group such as 1-homopiperidinyl, etc.

The substituted alkyl group, the substituted cycloalkyl group, the substituted alkenyl group, and the substituted alkynyl group have one or more substituents which are the same or different, and the substituents includes, for example, a halogen atom, a cyano group, a phenoxy group, a benzyloxy group, a trifluoromethyl group, a hydroxy group, a lower alkoxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a lower alkoxycarbonylamino group, a carboxyl group, a lower alkoxycarbonyl group, a lower alkylthio group, a lower alkylsulfinyl group, a lower alkylsulfonyl group, a lower alkanoylamino group, a lower alkylsulfonamido group, a phthalimido group, a heteroaryl group, or a group of the formula: ―NR⁴¹R⁵¹ (R⁴¹ and R⁵¹ are as defined above).

The substituted alkyl group includes an alkyl group having 1 to 6 carbon atoms which is substituted by a substituted or unsubstituted cycloalkyl group, or an aralkyl group or a substituted aralkyl group.

The aralkyl group includes an alkyl group having 1 to 6 carbon atoms which is substituted by the above-mentioned aryl group, for example, benzyl, 1-phenylethyl, 2-phenylethyl, 2-naphthylmethyl, etc:

The preferable groups for Y are, for example, a substituted or unsubstituted phenyl group, or a substituted or unsubstituted pyridyl group. The substituted phenyl group and the substituted pyridyl group have one or more substituents which are the same or different, and the preferable substituents are, for example, a halogen atom such as fluorine atom, chlorine atom, etc., a cyano group, a trifluoromethyl group, a nitro group, a hydroxy group, a methylenedioxy group, a lower alkyl group, a lower alkoxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a carboxyl group, a lower alkoxycarbonyl group, a lower alkylthio group, a lower alkylsulfinyl group, a lower alkylsulfonyl group, a lower alkanoylamino group, a lower alkylsulfonamido group, or a group of the formula: ―M¹―E¹―T (M¹, E¹ and T are as defined above).

The preferable groups for M¹ are, for example, a direct bond or an oxygen atom.

The preferable groups for E¹ are, for example, a straight alkylene, alkenylene or alkynylene chain having 1 to 6 carbon atoms, and the more preferable ones are a straight alkylene or alkynylene chain having 1 to 3 carbon atoms.

The preferable groups for T are, for example, a hydroxy group, a cyano group, a lower alkoxy group, a lower alkanoyloxy group, a lower alkanoylamino group, a heteroaryl group, or a group of the formula: ―NR⁴¹R⁵¹ (R⁴¹ and R⁵¹ are as defined above), and the more preferable one is a heteroaryl group such as 2-pyridyl, 3-pyridyl, 4-pyridyl, 2-methyl-3-pyridyl, 1-imidazolyl, 1,2,4-triazol-1-yl, etc., or a group of the formula: ―NR⁴¹R⁵¹.

The preferable group of the formula: ―NR⁴¹R⁵¹ includes, for example, dimethylamino, diethylamino, diisopropylamino, 1-pyrrolidinyl, 1-piperidinyl, morpholino, 4-methylpiperidinyl, etc.

The more preferable groups for Y are, for example, a phenyl group being substituted by a lower alkyl group or a lower alkoxy group, or a pyridyl group being substituted by a lower alkyl group or a lower alkoxy group.

The preferable groups for R¹ are, for example, a hydrogen atom, a substituted or unsubstituted alkyl group, or a substituted or unsubstituted alkenyl group. The substituted alkyl group and the substituted alkenyl group have one or more substituents which are the same or different, and the preferable substituents are, for example, a halogen atom such as fluorine atom or chlorine atom, a cyano group, a benzyloxy group, a hydroxy group, a lower alkoxy group, a lower alkanoyloxy group, a carbamoyl group, a lower alkylaminocarbonyl group, a di-lower alkylaminocarbonyl group, a carboxyl group, a lower alkoxycarbonyl group, a lower alkylthio group, a lower alkylsulfinyl group, a lower alkylsulfonyl group, an aryl group, a lower alkanoylamino group, a lower alkylsulfonamido group, a phthalimido group, or a heteroaryl group. The more preferable substituents are, for example, a fluorine atom, a chlorine atom, a cyano group, a hydroxy group, a lower alkoxy group, a carbamoyl group, a 2-pyridyl group, a 3-pyridyl group, a 4-pyridyl group, etc. The further more preferable substituents for R¹ are, for example, an unsubstituted alkyl or alkenyl group.

The preferable groups for R² are, for example, a hydrogen atom, a methyl group, an ethyl group, a propyl group, or an isopropyl group. The preferable groups for R³ are, for example, an isopropyl group or a tert-butyl group.

The preferable groups for D¹ are, for example, a methylene group or an ethylene group. The preferable groups for Q are, for example, a hydroxy group, a substituted or unsubstituted heteroaryl group, or a group of the formula: ―NR⁴R⁵ (N⁴ and R⁵ are as defined above). The more preferable groups are, for example, a hydroxy group, a 1-pyrazolyl group, a 3,5-dimethyl-1-pyrazolyl group, a 1-imidazolyl group, a 2-methyl-1-imidazolyl group, a 1,2,4-triazol-1-yl group, a 1-piperidinyl group, a 1-pyrrolidinyl group, a 4-methyl-1-piperazinyl group, a morpholino group, a diethylamino group or a dipropylamino group.

The preferable groups for D² are, for example, a direct bond, a methylene group or an ethylene group.

The preferable groups for M are, for example, an oxygen atom, or a group of the formula: ―NHC(=O)―, ―C(=O)NH―, or ―NR⁶―.

The preferable groups for E are, for example, methylene, ethylene or trimethylene.

The preferable groups for W are, for example, a hydroxy group, a substituted or unsubstituted heteroaryl group, or a group of the formula: ―NR⁴R⁵. The more preferable groups are, for example, a hydroxy group, a 2-pyridyl group, a 3-pyridyl group, a 4-pyridyl group, a 1-pyrazolyl group, a 3,5-dimethyl-1-pyrazoyl group, a 1-imidazolyl group, a 2-methyl-1-imidazolyl group, a 1,2,4-triazol-1-yl group, a 1-piperidinyl group, a 1-pyrrolidinyl group, a 4-methyl-1-piperazinyl group, a morpholino group, a diethylamino group, or a dipropylamino group.

The preferable groups represented by the following formula (2): 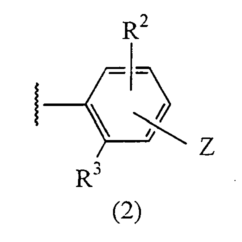 are, for example 2,6-diisopropyl-4-(2-pyridylmethoxy)phenyl group, 2,6-diisopropyl-4-(3-pyridylmethoxy)phenyl group, 2,6-diisopropyl-4-(4-pyridylmethoxy)phenyl group, 2,6-diisopropyl-4-{2-(1-piperidinyl)-ethoxy}phenyl group, 2,6-diisopropyl-4-(3-(1-piperidinyl)propoxy)phenyl group, 2,6-diisopropyl-4-{2-(1-pyrrolidinyl)ethoxy}phenyl group, 2,6-diisopropyl-4-{2-(morpholino)ethoxy}phenyl group, 2,6-diisopropyl-4-{2-(4-methyl-1-piperazinyl)ethoxy}phenyl group, 2,6-diisopropyl-4-{2-(1,2,4-triazol-1-yl)ethoxy}phenyl group, 2,6-diisopropyl-4-{3-(1,2,4-triazol-1-yl)propoxy}phenyl group, 2-tert-butyl-5-hydroxymethylphenyl group, 2-tert-butyl-5-(1-pyrazolyl)-methylphenyl group, 2-tert-butyl-5-{2-(1-pyrazolyl)ethyl}phenyl group, 2-tert-butyl-5-(3,5-dimethyl-1-pyrazolyl)methylphenyl group, 2-tert-butyl-5-(1-imidazolyl)methylphenyl group, 2-tert-butyl-5-{2-(1-imidazolyl)ethyl}-phenyl group, 2-tert-butyl-5-(2-methyl-1-imidazolyl)-methylphenyl group, 2-tert-butyl-5-(1,2,4-triazol-1-yl)methylphenyl group, 2-tert-butyl-5-{2-(1,2,4-triazol-1-yl)-ethyl}-phenyl group, 2-tert-butyl-5-(1-piperidinyl)methylphenyl group, 2-tert-butyl-5-(1-pyrrolidinyl)methyl-phenyl group, 2-tert-butyl-5-(4-methyl-1-piperazinyl)methylphenyl group, 2-tert-butyl-5-morpholinomethylphenyl group, 2-tert-butyl-5-diethylaminomethylphenyl group, 2-tert-butyl-5-dipropylaminomethylphenyl group, 2-tert-butyl-5-(2-pyridyl)methylaminomethylphenyl group, 2-tert-butyl-5-(3-pyridyl)-methylaminophenyl group, 2-tert-butyl-5-(4-pyridyl)-methylaminomethylphenyl group, 2-tert-butyl-5-{N-(2-pyridyl)-methyl-N-methyl}-aminomethylphenyl group, 2-tert-butyl-5-{N-(3-pyridyl)-methyl-N-methyl}aminomethylphenyl group, or 2-tert-butyl-5-{N-(4-pyridyl)-methyl-N-methyl}aminomethylphenyl group.

The "prodrug" includes a compound which can easily be hydrolyzed in the living body, and can reproduce the compound of the formula (1) or (51). The "prodrug" is, for example, when such a compound of the formula (1) or (51) has a carboxyl group, then ones wherein said carboxyl group is replaced by an alkoxycarbonyl group, an alkylthiocarbonyl group, or an alkylaminocarbonyl group, or when a compound of the formula (1) or (51) has an amino group, then ones wherein said amino group is substituted by an alkanoyl group to form an alkanoylamino group, or substituted by an alkoxycarbonyl group to form an alkoxycarbonylamino group, or converted to an acyloxymethylamino group or a hydroxyamine. When a compound of the formula (1) or (51) has a hydroxy group, the prodrug thereof is, for example, compounds wherein said hydroxy group is substituted by an acyl group as mentioned above and converted to an acyloxy group, or converted to a phosphate ester, or converted to an acyloxymethyloxy group. The alkyl moiety of groups being used for making a prodrug may be the above-mentioned alkyl groups, and said alkyl group may optionally be substituted, for example, by an alkoxy group having 1 to 6 carbon atoms, etc. The preferable example are, for example, in the compounds wherein a carboxyl group is converted to an alkoxycarbonyl group, a lower (e.g., having 1 to 6 carbon atoms) alkoxycarbonyl group such as methoxycarbonyl, ethoxycarbonyl, or a lower (e.g., having 1 to 6 carbon atoms) alkoxycarbonyl group being substituted by an alkoxy group such as methoxymethoxycarbonyl, ethoxymethoxycarbonyl, 2-methoxyethoxycarbonyl, 2-methoxyethoxymethoxycarbonyl, or pivaloyloxymethoxycarbonyl.

The acid for forming an acid addition salt includes, for example, inorganic acids such as hydrochloric acid, hydrobromic acid, hydroiodic acid, sulfuric acid, nitric acid, etc., or organic acids such as acetic acid, oxalic acid, citric acid, malic acid, tartaric acid, fumaric acid, maleic acid, methanesulfonic acid, benzenesulfonic acid, etc.

When the HMG-CoA reductase inhibitor etc. or the compound of a formula (1) etc. have a carboxyl group, etc., then the present compounds may be in the form of a salt with an organic base such as diethanolamine salt, ethylenediamine salt, or N-methyl-glucamine salt, or a salt with an alkaline earth metal such as calcium salt or magnesium salt, or a salt with an alkali metal such as lithium salt, potassium salt, or sodium salt.

The HMG-CoA reductase inhibitor etc. or a compound of a formula (1) etc. may have a stereoisomer due to an asymmetric carbon atom thereof. In such cases, the HMG-CoA reductase inhibitor etc. or a compound of a formula (1) etc. also include a mixture of the isomers or each isomer.

The HMG-CoA reductase inhibitor etc., ezetimibe etc., or a compound of a formula (1) etc. may be in the form of an anhydrous product thereof, or in the form of a solvate thereof such as hydrate.

The HMG-CoA reductase inhibitors etc. and the preparations of the same is well known to the persons skilled in the art. For example, it is disclosed in the specification of US 4,346,227 for pravastatin, US 4,450,171 for simvastatin, US 4,231,938 for lovastatin, US 4,739,073 for fluvastatin, US 5,273,995 for atorvastatin, Japanese Patent Pabulication No. JP H05-178841 for rosuvastatin, and JP H01-279866 for pitavastatin respectively.

The ezetimibe etc. and the preparations of the same are disclosed in US 5,846,966 for example.

The compound of formula (1) etc. and the preparations of the same are well known to the person skilled in the art. For example, they are disclosed in the above Patent References 1 (International publication No. 00/09505 pamphlet).

The pharmaceutical composition of the present invention comprises, in combination of (A) HMG-CoA reductase inhibitor etc. and/or ezetimibe etc., with (B) compounds of formula (1) etc., and the pharmaceutical composition may be accept so long as (A) HMG-CoA reductase inhibitors etc. and/or ezetimibe etc., and (B) the compounds of formula (1) etc. can be combined when administered. Thus, the pharmaceutical composition of the present invention may be either a single preparation obtained by formulating the (A) HMG-CoA reductase inhibitor etc. and/or ezetimibe etc., with (B) the compound of formula (1) etc. simultaneously, or a combination of at least two of a preparation obtained by formulating (A) HMG-CoA reductase inhibitor etc. and/or ezetimibe etc., and (B) the compound of formula (1) etc. separately, as long as (A) HMG-CoA reductase inhibitor etc. and/or ezetimibe etc., and (B) the compound of formula (1) etc. can be combined when administered.

The dosage form is not limited to specific ones and includes for example, (a) a composition comprising (A) HMG-CoA reductase inhibitor etc. and/or ezetimibe etc. and (B) a compound of formula (1) etc., that is, a dosage form of a single preparation; (b) a dosage form for simultaneous administration via same route of two types of preparations obtained by formulating (A) HMG-CoA reductase inhibitor etc. and/or ezetimibe etc., and (B) the compound of formula (1) etc. separately; (c) a dosage form for an time-lagged administration via same route of two types of preparations obtained by formulating (A) HMG-CoA reductase inhibitor etc. and/or ezetimibe etc., and (B) the compound of formula (1) etc. separately (for example, the order of administration with (A) HMG-CoA reductase inhibitor etc. and/or ezetimibe etc., followed by administration with (B) the compound of formula (1) etc., or the reverse order of administration of the same); (d) a dosage form for simultaneous administration via different routes of two types of preparations obtained by formulating (A) HMG-CoA reductase inhibitor etc. and/or ezetimibe etc., and (B) the compound of formula (1) etc. separately; (e) a dosage form for an time-lagged administration via different routes of two types of preparations obtained by formulating (A) HMG-CoA reductase inhibitor etc. and/or ezetimibe etc., and (B) the compound of formula (1) etc. separately (for example, the order of administration with (A) HMG-CoA reductase inhibitor etc. and/or ezetimibe etc., followed by administration with (B) the compound of formula (1) etc., or the reverse order of administration of the same).

For the time-tagged administration, it is necessary for both of (A) HMG-CoA reductase inhibitor etc. and/or ezetimibe etc. and (B) the compound of formula (1) etc. to coexist within the body for an enough time to potentiate the blood cholesterol lowering action.

In this invention, the combination ratio of (A) HMG-CoA reductase inhibitor etc. and (B) the compound of formula (1) etc. is generally in the range of 1:100 to 100:1, preferably 1:10 to 10:1, more preferably 1:5 to 5:1 1 by weight, either in a single preparation or a separate preparations. Besides, the combination ratio of ezetimibe etc. and the compound of formula (1) etc. is generally in the range of 1:100 to 100:1, preferably 1:10 to 10:1, more preferably 1:5 to 5:1 by weight, either in a single preparation or separate preparations.

The HMG-CoA reductase inhibitor etc., ezetimibe etc. and/or the compound of formula (1) etc. can be administered either parenterally or orally when used as the above-mentioned pharmaceutical compositions etc. [1] to [12]. That is, these pharmaceutical compositions etc. can be formulated in liquid preparations such as solutions, emulsions, suspensions, etc., which can be administered by injection, and if necessary, buffering agents, solubilizers and isotonic agents may be added thereto. The present compounds can also be administered rectally in the form of a suppository. The present compounds can also be administered orally in the form of a conventional dosage form such as tablets, capsules, syrups, and suspension. These dosage forms can be formulated by mixing an active ingredient with conventional carriers or excipients or binding agents or stabilizers in a conventional manner.

The dosage and the frequency of administration of the HMG-CoA reductase inhibitor etc., ezetimibe etc., and/or the compound of formula (1) etc. may vary according to the conditions, ages, weights of the patients and the dosage form, etc., but each of the HMG-CoA reductase inhibitor etc., ezetimibe etc. or the compound of formula (1) etc. can usually be administered orally in a dose of 1 to 500 mg per day in adult, once a day, or divided into 2 to 4 dosage units.

This invention includes a commercial package which comprises a combination of (A) HMG-CoA reductase inhibitor etc. and/or ezetimibe etc. with (B) the compound of formula (1) etc., and a package insert indicating that said combination may be used or should be used for lowering of the blood cholesterol; a commercial package which comprises a pharmaceutical composition comprising HMG-CoA reductase inhibitor etc. and/or ezetimibe etc., and a package insert indicating that said pharmaceutical composition may be used or should be used for potentiating a blood cholesterol lowering action with a compound of the formula (1) etc.; and a commercial package which comprises (A) a pharmaceutical composition comprising a compound of formula (1) etc. and (B) a package insert indicating that said pharmaceutical composition may be used or should be used for potentiating a blood cholesterol lowering action with a HMG-CoA reductase inhibitor etc. and/or ezetimibe etc.

The effect of the preparation of the present invention is described specifically in the following examples, but is not limited to the embodiment.

### Example 1

Effects of LDL binding, uptake and degradation activity by a combination of a compound of formula (1) and atorvastatin in a cultured human hepatic cell line.

### (Method)

The cultured human hepatic cell line HepG2 cell was purchased from the Dainippon Pharmaceuticals Co. Ltd. (Osaka, Japan). The determinations of LDL binding, uptake and degradation activity were performed according to a prior report (Method in Enzymology, 98, 241-255, 1983). Cells were seeded in a 24 well plate and were incubated in the Dulbecco's modified Eagle/F-12 (DMEM/F-12) medium containing 10% fetal bovine serum and antibiotics at 37°C for two days. After washing the cells, a DMEM/F-12 medium containing 10% lipoprotein-removed serum, the test substrate and the antibiotics was added and the cells were incubated at 37°C for 19 hours. After adding [¹²⁵I]LDL, the cells were incubated at 37°C for another 5 hours. The culture supernatant was collected and tricloroacetic acid was added thereto, and then it was statically kept for 30 minutes or more at 4°C. After centrifuging (700xg, 4°C, 15min.), the supernatant was separated and sodium iodide was added thereto, and further hydrogen peroxide was added. After treating the mixture at room temperature for 5 min., chloroform was added and the mixture was stirred. After centrifuging (700×g, 4°C, 5min.), the radioactivity in the supernatant was measured by a gamma counter, and then the data thus obtained was evaluated as an amount of degradation of LDL. Separately, after washing the cells thoroughly, a dissociation buffer (50mM NaCl, 10mM 2-[4-(2-hydroxyethyl)-1-piperadinyl]ethane sulfonic acid (HEPES, pH 7.4), 10mg/mL heparin) was added and then the cells were statically kept at 4°C for 60 min. or more. The dissociation buffer was recovered and the radioactivity was determined by a gamma counter and this data was evaluated as an amount of binding of LDL. Furthermore, 1N sodium hydroxide was added to the remaining cells and the mixture was reacted for more than 10 min. to dissolve the cells. This cell lysate was recovered and the radioactivity was measured by the gamma counter and then this data was evaluated as an amount of uptake of LDL. On the other hand, the amounts of non-specific degradation, binding and uptake were evaluated by coexisting an excess of non-labeled LDL on addition of [¹²⁵I] LDL, while the amounts of specific degradation, binding and uptake were obtained by subtracting the amounts of non-specific degradation, binding and uptake from the total amounts of degradation, binding and uptake and further calibrating with the amounts of protein. For test substate, N-[1-butyl-4-[3-[3-(hydroxy)propoxy]phenyl]-1,2-dihydro-2-oxo-1,8-naphthyridine-3-yl]-N'-(2,6-diisopropyl-4-aminophenyl) urea (hereinafter, referred to as compound A) and atorvastatin were used.

### (Results)

As showed in table 1, the compound A (0.01 to 1µM) and atorvastatin (0.1 to 10µM) increased the amounts of binding, uptake and degradation of LDL and thus this demonstrated the enhancement of the function of LDL receptor. Additionally, the combination of atorvastatin (1µM) and compound A (0.01 to 1µM) showed the significantly higher effect on increase of the amounts of binding, uptake, and degradation of LDL than the case of treatments with atorvastatin (10µM) alone or the compound A (1µM) alone.

It is notable that the increments of the amounts of binding, uptake and degradation were decreased with increase of the concentration of the compound A by 10 folds (such as an order of 0.01, 0.1 and 1 µM), and also a similar tendency was observed with increase of the concentration of atorvastatin by 10 folds (such as order of 0.1, 1 and 10 µM), while the combination of them showed the remarkable increase of the amounts of binding, uptake and degradation. It is not usual that an addition of other agent exhibits remarkably increased effect where increase of the dosage of a particular single preparation results in decrease of the incremental rate of effects. Accordingly, such effects of the above combination would not be predictable by a person skilled in the art from the technical knowledge in the art. Thus, the preparation of this invention (including the preparations for a combination therapy) exploits a possibility of the new therapy.

**Table 1**

| Treated group | Conc. | (µM) | Binding Amounts (ng/ mg protein) | Uptake Amounts (ng/ mg protein) | Degradation Amounts (ng/ mg protein) |
|---|---|---|---|---|---|
| | Compd.A | Atorvastatin | | | |
| Control | ― | ― | 72.0 ± 2.7 | 780.3 ± 16.3 | 677.6 ± 37.2 |
| Compd. A | 0.01 | ― | 85.2 ± 3.6* | 852.9 ± 47.3 | 927.5 ± 90.0 * |
| | 0.1 | ― | 90.9 ± 4.1* | 886.4 ± 29.2 | 1016.8 ± 45.3* |
| | 1 | ― | 93.7 ± 2.5* | 1172.2 ± 46.2* | 1126.3 ± 70.3* |
| Atorvastatin | ― | 0.1 | 81.7 ± 2.1 | 1043.4 ± 16.7* | 800.9 ± 32.5 |
| | ― | 1 | 86.3 ± 2.7* | 1033.4 ± 52.8* | 1000.5 ± 62.9 * |
| | ― | 10 | 95.5 ± 4.0* | 1145.0 ± 50.9* | 1062.3 ± 66.0* |
| Compd. A +Atorvastatin | 0.01 | 1 | 98.5 ± 1.8* | 1234.1 ± 16.7* | 1248.5 ± 91.6* |
| | 0.1 | 1 | 104.2 ± 2.8* | 1306.2 ± 29.4* | 1402.2 ± 78.8* |
| | 1 | 1 | 110.8 ± 0.9* | 1443.8 ± 20.2* | 1534.0 ± 54.6* |

| | | | | | |
|---|---|---|---|---|---|
| The values in the above table represent the mean ± standard error (number of experiment: 6). * represents significant difference from control (*P<0.05; Dunnett test or Steel test). | | | | | |

### Example 2

Effects of a blood cholesterol lowering action of a combination of a compound A and atorvastatin in an endogenous hyperlipemia rabbit

### (Method)

8 weeks old male NewZealand White rabbit were bred for 12 days with ad libitum water and feeding conditions (rabbit · guinea pig breeding feed RC4, Oriental Yeast Co., Ltd., Tokyo, Japan). Thereafter, the diet was replaced with the casein diet containing 27% casein (Oriental Yeast Co., Ltd., Tokyo, Japan), and then the restricted feeding to the 100g per 3kg weight was performed for one week. At this point, the blood was collected via auricular veins and the plasma total cholesterol amounts was measured and then these rabbits were divided such that the mean of the weight and the plasma total cholesterol were equal approximately. Thereafter, continuing the restricted feeding of casein diet, the each agent was administered in the following conditions for 3 weeks: compound A (1mg/kg), atorvastatin (1 and 3 mg/kg), a combination of compound A (1mg/kg) and atorvastatin (1mg/kg), and a combination of compound A (1mg/kg) and atorvastatin (3mg/kg). Each agent was dissolved in propylene glycol containing 0.5% polyoxyethylenesorbitan monooleate (Tween 80) and then these were administered orally. On the other hand, for control group, the solvent (propylene glycol comprising 0.5% Tween 80) was administered orally. At one day after final administration, the blood was collected and plasma total cholesterol was measured and also the lipoprotein was fractioned according to the differential ultracentrifugation method (Nobuhiro, Yamada, Yasushi Sato edit.: Study strategy of Arteriosclerotic+Hyperlipemia, 63-69, 1996, Shujunsha Co. Ltd.), and then the amounts of cholesterol in the fraction of LDL were measured. The amount of total cholesterol in plasma and the amount of cholesterol in LDL fraction were determined by using an automatic biochemical analyzer (Biochemical Analyzer CHEM 1, Bayer Corporation, NY, USA).

### (Results)

As showed in Table 2, in rabbit with endogenous hyperlipemia caused by a casein diet load, compound A (1mg/kg) showed significant effects on plasma total cholesterol lowering action and plasma LDL cholesterol lowering action, and atorvastatin (1 and 3 mg/kg) also showed the similar tendency of lowering of plasma total cholesterol and plasma LDL cholesterol. A combination of compound A (1mg/kg) and atorvastatin (1 and 3 mg/kg) showed more potent effect on plasma lipid lowering action than the case of the single agent and also the group of these combination showed significant effect on plasma total cholesterol lowering action compared to the case of the group of atorvastatin 3mg/kg. While the group administered with atorvastain 1mg/kg did not show significant effects on plasma total cholesterol lowering action and plasma LDL cholesterol lowering action compared to the control group, the group administered with compound A 1mg/kg+atorvastatin 1mg/kg showed the significant effect on plasma total cholesterol lowering action even compared to the group administered with the compound A 1mg/kg. Thus this will be one example that a combination of both compounds exhibits the remarkable increase of the actions.

**Table 2**

| Treated group | Dosage (mg/kg) | number of experiment | plasma total cholesterol (mg/dl) | plasma LDL cholesterol (mg/dl) |
|---|---|---|---|---|
| Control | - | 7 | 172 ± 20 | 100 ± 13 |
| Comp. A | 1 | 7 | 108 ± 5* | 52 ± 9* |
| Atorvastatin | 1 | 6 | 115 ± 18 | 62 ± 18 |
| Atorvastatin | 3 | 7 | 122 ± 15 | 59 ± 16 |
| Comp. A | 1 | 7 | 64 ± 17^{††,‡,§} | 50 ±21 |
| + Atorvastatin | 1 | | | |
| Comp. A | 1 | 7 | 70 ± 17^{††,§} | 38 ± 16† |
| + Atorvastatin | 3 | | | |

| | | | | |
|---|---|---|---|---|
| The values in the above table represent the mean ± standard error. *, † and †† represents significant difference from control (*P<0.05; Student t test or Welch test, tP<0.05; ^{††}P<0.01; Dunnett test). ‡ represents significant difference from the group of compound A 1mg/kg (^{‡}P<0.05; Welch test) and § represents significant difference from the group of atorvastatin 3mg/kg (^{§}P<0.05; Student t test). | | | | |

### Example 3

Effects of an expression of LDL receptor protein in liver by a combination of a compound A and atorvastatin in an endogenous hyperlipemia rabbit

### (Method)

For the rabbit used in Example 2, liver was taken out after one day of a final administration of the agent. A homogenizing buffer (20mM Tris-HCl (pH 8.0), 1mM CaCl₂, 150mM NaCl, a mix of protease inhibitor) was added to a part of this liver and the mixture was homogenized and centrifuged at 8000×g, 4°C for 10 min., thereafter the supernatant was ultracentrifuged at 10,000×g, 4°C for 60 min. The resulting precipitate was washed by adding the homogenizing buffer and centrifuging at 10,000×g, 4°C for 30 min., and thereafter was suspended in a suspension buffer (125mM Tris-maleate (pH 6.8), 1mM CaCl₂, 160 mM NaCl, 1% (w/v) Triton X-100, Protease inhibitor (Complete™, Boehringer Ingelhaim Co., Ltd., Ingelheim, Germany)) to prepare a fraction of the liver membrane. After mixing with a buffer (125mM Tris-HCl (pH 6.8), 20% (v/v) glycerol, 4% (w/v) sodium dodecyl sulfate (hereinafter, referred to as SDS) and 0.001% (w/v) bromophenol blue), the fraction was subjected to SDS polyacrylamide electrophoresis (using a 7.5% polyacrylamide gel), and the resultant was transferred to a polyvinylidene difluoride (PVDF) membrane and then the amount of LDL receptor protein was evaluated by an immunoblotting using anti-LDL receptor antibody. The amount of binding with antibody was visualized by chemiluminescence technique using X ray film and then the shading of the band of LDL receptor was digitized using a scanner and image software.

### (Results)

As showed in Figure 1, in a rabbit with endogenous hyperlipemia caused by casein diet load, compound A (1mg/kg) and atorvastatin (1 and 3 mg/kg) increased the amount of the expression of LDL receptor protein in liver. A combination of compound A (1mg/kg) and atorvastatin (1 and 3 mg/kg) showed the more potent effect on the increase of the expression of LDL receptor protein compared to the case of a single agent. Thus, it is confirmed that, even in vivo, a combination of both compounds show a remarkable increase of the actions.

### Example 4

The following components 1 to 5 are mixed and then wet-granulated with an aqueous solution of component 6 and then are mixed with component 7. The resulting mixture is compressed to give a 120mg tablet.

| | | |
|---|---|---|
| 1. | Compound A | 5mg/tablet |
| 2. | pravastatin | 5mg/tablet |
| 3. | 3. lactose | 72.5mg/tablet |
| 4. | corn starch | 30mg/tablet |
| 5. | calcium carboxymethylcellulose | 5mg/tablet |
| 6. | hydroxypropylcellulose (HPC-L) | 2mg/tablet |
| 7. | magnesium stearate | 0.5mg/tablet |

### Example 5

The following components 1 to 5 are mixed and then wet-granulated with an aqueous solution of component 6 and then are mixed with component 7. The resulting mixture is compressed to give a 120mg tablet.

| | | |
|---|---|---|
| 1. | Compound A | 5mg/tablet |
| 2. | ezetimibe | 5mg/tablet |
| 3. | lactose | 72.5mg/tablet |
| 4. | corn starch | 30mg/tablet |
| 5. | calcium carboxymethylcellulose | 5mg/tablet |
| 6. | hydroxypropylcellulose (HPC-L) | 2mg/tablet |
| 7. | magnesium stearate | 0.5mg/tablet |

### Example 6

(1) The following components 1 to 4 are mixed and then wet-granulated with an aqueous solution of component 5 and then are mixed with component 6. The resulting mixture is compressed to give a 120mg tablet.

| | | |
|---|---|---|
| 1. | Compound A | 10mg/tablet |
| 2. | lactose | 72.5mg/tablet |
| 3. | corn starch | 30mg/tablet |
| 4. | calcium carboxymethylcellulose | 5mg/tablet |
| 5. | hydroxypropylcellulose (HPC-L) | 2mg/tablet |
| 6. | magnesium stearate | 0.5mg/tablet |

(2) The above tablet (1) and a medicinal compositions of ezetimibe (10mg) and simvastatin (10, 20, 40, 80mg) (Commercial Name: VYTORIN, Merck/Schering-Plough Pharmaceuticals) are simultaneously administered to the patients suffered from hyperlipemia or arteriosclerosis.

It is easily predictable that there should be many cases in which targeted level for therapy is not achieved based on the usage of ezetimibe (10mg per 1 dose), a currently marketed agent in U. S., because the degree of LDL cholesterol lowering action by a single agent of ezetimibe (as above mentioned) is only by 15 to 20% and further this value cannot be reduced even if the dosage is increased as reported in European Atherosclerosis Society Congress, 2002.

On the other hand, compound A as an ACAT inhibitor is expected to reduce the lipid in blood due to an inhibitory action of absorption of cholesterol from an intestinal tract and an inhibitory action of VLDL secretion in liver and furthermore can increase the expression and activity of LDL receptor even if it is used alone, as shown in Examples 1 to 3. Therefore a composition and a combination comprising ezetimibe (which can reduce lipid due to the inhibitory action of absorption of cholesterol in an intestinal tract) and compound A (and/or HMG-CoA reductase inhibitor) can provide a remarkably effective therapy for lowering the LDL cholesterol than the case of a single agent.

### INDUSTRIAL APPLICABILITY

This invention provide the method for elevating the expression of LDL receptor in more safety and higher degree compared with the case of a single agent of HMG-CoA reductase inhibitor etc. ezetimibe etc. or a compound of formula (1) etc.. This invention also provide an agent for hyperlipidemia or arteriosclerosis comprising (A) HMG-CoA reductase inhibitor etc. and/or ezetimibe etc. and (B) a compound of formula (1) etc.; an agent for hyperlipidemia or arteriosclerosis comprising any one of (A) or (B), which is to be used together with another one; a pharmaceutical composition for potentiating blood cholesterol lowering action, which comprises either one of (A) or (B), which is to be used in the therapy of said disease with a pharmaceutical composition comprising another one, as a therapeutic agent therefor.

## Claims

1. An agent for treating hyperlipidemia or arteriosclerosis comprising
(A) 3-hydroxy-3-methylglutaryl coenzyme A reductase inhibitor or a prodrug thereof or a pharmaceutically acceptable salt of the same and/or ezetimibe or a prodrug thereof or a pharmaceutically acceptable salt of the same; and
(B) a compound of the formula (1): 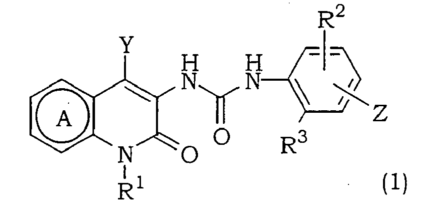
wherein Ring A is a substituted or unsubstituted pyridine ring;
Y is a substituted or unsubstituted alkyl group, a substituted or unsubstituted cycloalkyl group, or a substituted or unsubstituted aromatic group;
R¹ is a hydrogen atom, a substituted or unsubstituted alkyl group, a substituted or unsubstituted alkenyl group, a substituted or unsubstituted alkynyl group, or a substituted or unsubstituted cycloalkyl group;
R² is a hydrogen atom or a lower alkyl group;
R³ is a lower alkyl group;
Z is a group represented by either of the following formula 1) or 2):
1) ―D¹―Q
wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸―(R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or
2) ―D²―M―E―W
wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶―(R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond,
or a prodrug thereof, or a pharmaceutically acceptable salt of the same.

2. The agent for hyperlipidemia or arteriosclerosis according to claim 1, wherein in the formula (1), Ring A is one of the groups of the following formulae (a), (b) and (c): 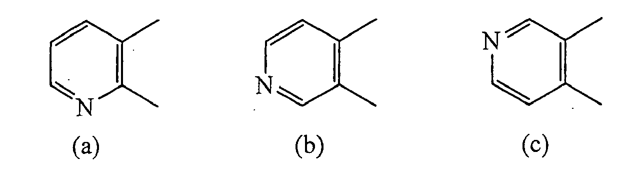
Y is a substituted or unsubstituted aromatic group;
R¹ is a substituted or unsubstituted alkyl group, or a substituted or unsubstituted alkenyl group;
Z is a group of the formula: ―D¹―Q, wherein the D¹ is a direct bond, Q is a hydroxy group or a group of the formula: ―NR⁴R⁵.

3. The agent for hyperlipidemia or arteriosclerosis according to claim 1 or 2, wherein the compound of formula (1) is represented by the formula (51): 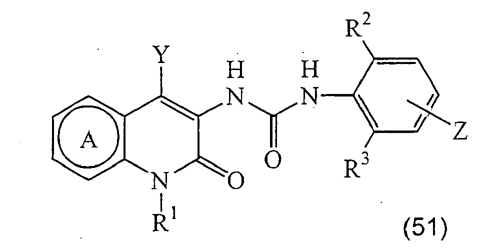 wherein the Ring A, R¹, R², R³ and Z have the same meanings as defined in claim 1; Y is a phenyl group substituted by a group represented by the formula ―M¹―E¹―T, wherein M¹ is an oxygen atom, E¹ is a hydrocarbon group having 2 to 4 carbon atoms, T is a hydroxy group or a group represented by the formula ―NR⁴¹R⁵¹ (R⁴¹ and R⁵¹ are independently a hydrogen atom, a lower alkoxy-substituted or unsubstituted lower alkyl group, a cycloalkyl group, a lower alkoxycarbonyl group, or an aralkyl group, or alternatively R⁴¹ and R⁵¹ may combine each other, and with the adjacent nitrogen atom to which they bond, form a saturated cyclic amino group having 4 to 8 carbon atoms as ones forming the said ring, and optionally having one ―NR⁸¹― (R⁸¹ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof.

4. The agent for hyperlipidemia or arteriosclerosis according to claim 1, wherein the compound of formula (1) is N-[1-butyl-4-[3-[3-(hydroxy)propoxy]phenyl]-1,2-dihydro-2-oxo-1,8-naphthyridin-3-yl]-N'-(2,6-diisopropyl-4-aminophenyl)urea.

5. The agent for hyperlipidemia or arteriosclerosis according to any one of claims 1 to 4, wherein 3-hydroxy-3-methylglutaryl coenzyme A reductase inhibitor is selected from the group consisting of pravastatin, simvastatin, lovastatin, fluvastatin, atorvastatin, rosuvastatin, and pitavastatin.

6. An agent for hyperlipidemia or arteriosclerosis comprising a compound of the formula (1): 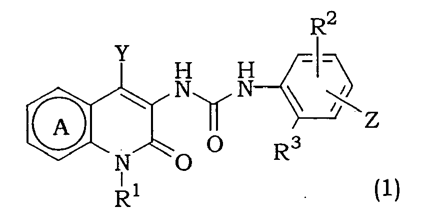 wherein Ring A is a substituted or unsubstituted pyridine ring;
Y is a substituted or unsubstituted alkyl group, a substituted or unsubstituted cycloalkyl group, or a substituted or unsubstituted aromatic group;
R¹ is a hydrogen atom, a substituted or unsubstituted alkyl group, a substituted or unsubstituted alkenyl group, a substituted or unsubstituted alkynyl group, or a substituted or unsubstituted cycloalkyl group;
R² is a hydrogen atom or a lower alkyl group;
R³ is a lower alkyl group;
Z is a group represented by either of the following formula 1) or 2):
1) ―D¹―Q
wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸― (R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or
2) ―D²―M―E―W
wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶― (R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond,
or a prodrug thereof or a pharmaceutically acceptable salt of the same, to be used in combination with a pharmaceutical composition comprising 3-hydroxy-3-methylglutaryl coenzyme A reductase inhibitor or a prodrug thereof or a pharmaceutically acceptable salt of the same and/or ezetimibe or a prodrug thereof or a pharmaceutically acceptable salt of the same.

7. A pharmaceutical composition for potentiating a blood cholesterol lowering action to be used in a therapy using a pharmaceutical composition comprising 3-hydroxy-3-methylglutaryl coenzyme A reductase inhibitor or a prodrug thereof or a pharmaceutically acceptable salt of the same, and/or ezetimibe or a prodrug thereof or a pharmaceutically acceptable salt of the same,
which comprises a compound of the formula (1): 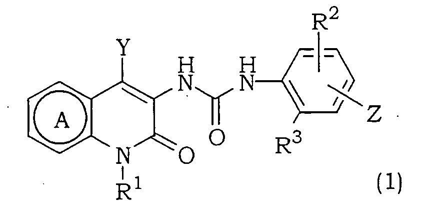 wherein Ring A is a substituted or unsubstituted pyridine ring;
Y is a substituted or unsubstituted alkyl group, a substituted or unsubstituted cycloalkyl group, or a substituted or unsubstituted aromatic group;
R¹ is a hydrogen atom, a substituted or unsubstituted alkyl group, a substituted or unsubstituted alkenyl group, a substituted or unsubstituted alkynyl group, or a substituted or unsubstituted cycloalkyl group;
R² is a hydrogen atom or a lower alkyl group;
R³ is a lower alkyl group;
Z is a group represented by either of the following formula 1) or 2):
1) ―D¹―Q
wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸― (R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or
2) ―D²―M―E―W
wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O) ―, ―C(=O)NH― or ―NR⁶― (R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond,
or a prodrug thereof or a pharmaceutically acceptable salt of the same.

8. An agent for treating hyperlipidemia or arteriosclerosis comprising 3-hydroxy-3-methylglutaryl coenzyme A reductase inhibitor or a prodrug thereof or a pharmaceutically acceptable salt of the same, and/or ezetimibe or a prodrug thereof or a pharmaceutically acceptable salt of the same, which is used in combination with a pharmaceutical composition comprising a compound of the formula (1): 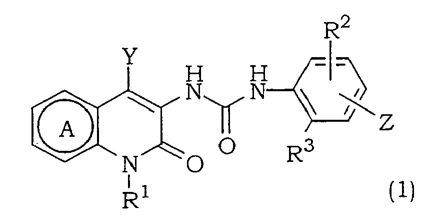 wherein Ring A is a substituted or unsubstituted pyridine ring;
Y is a substituted or unsubstituted alkyl group, a substituted or unsubstituted cycloalkyl group, or a substituted or unsubstituted aromatic group;
R¹ is a hydrogen atom, a substituted or unsubstituted alkyl group, a substituted or unsubstituted alkenyl group, a substituted or unsubstituted alkynyl group, or a substituted or unsubstituted cycloalkyl group;
R² is a hydrogen atom or a lower alkyl group;
R³ is a lower alkyl group;
Z is a group represented by either of the following formula 1) or 2):
1) ―D¹―Q
wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸―(R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or
2) ―D²―M―E―W
wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶―(R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond, or a prodrug thereof or a pharmaceutically acceptable salt of the same.

9. A pharmaceutical composition for potentiating a blood cholesterol lowering action comprising 3-hydroxy-3-methylglutaryl coenzyme A reductase inhibitor or a prodrug thereof or a pharmaceutically acceptable salt of the same, and/or ezetimibe or a prodrug thereof or a pharmaceutically acceptable salt of the same, which is used in a therapy using a pharmaceutical composition comprising a compound of the formula (1): 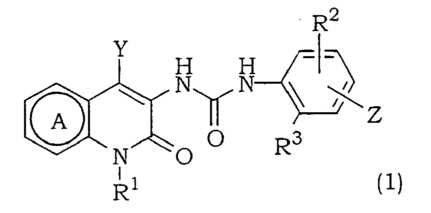 wherein Ring A is a substituted or unsubstituted pyridine ring;
Y is a substituted or unsubstituted alkyl group, a substituted or unsubstituted cycloalkyl group, or a substituted or unsubstituted aromatic group;
R¹ is a hydrogen atom, a substituted or unsubstituted alkyl group, a substituted or unsubstituted alkenyl group, a substituted or unsubstituted alkynyl group, or a substituted or unsubstituted cycloalkyl group;
R² is a hydrogen atom or a lower alkyl group;
R³ is a lower alkyl group;
Z is a group represented by either of the following formula 1) or 2):
1) ―D¹―Q
wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸―(R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or
2) ―D²―M―E―W
wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶―(R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond,
or a prodrug thereof or a pharmaceutical acceptable salt of the same.

10. A commercial package which comprises a pharmaceutical composition comprising a compound of the formula (1): 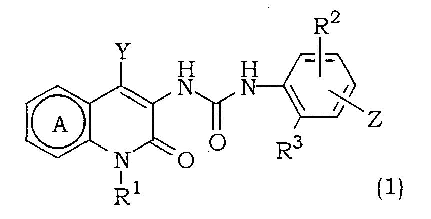 wherein Ring A is a substituted or unsubstituted pyridine ring;
Y is a substituted or unsubstituted alkyl group, a substituted or unsubstituted cycloalkyl group, or a substituted or unsubstituted aromatic group;
R¹ is a hydrogen atom, a substituted or unsubstituted alkyl group, a substituted or unsubstituted alkenyl group, a substituted or unsubstituted alkynyl group, or a substituted or unsubstituted cycloalkyl group;
R² is a hydrogen atom or a lower alkyl group;
R³ is a lower alkyl group;
Z is a group represented by either of the following formula 1) or 2):
1) ―D¹―Q
wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸―(R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or
2) ―D²―M―E―W
wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶―(R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond,
or a prodrug thereof or a pharmaceutically acceptable salt of the same, and a package insert indicating that said pharmaceutical composition may be used or should be used for potentiating a blood cholesterol lowering action with 3-hydroxy-3-methylglutaryl coenzyme A reductase inhibitor or a prodrug thereof or a pharmaceutically acceptable salt of the same, and/or ezetimibe or a prodrug thereof or a pharmaceutically acceptable salt of the same.

11. A commercial package which comprises a pharmaceutical composition comprising 3-hydroxy-3-methylglutaryl coenzyme A reductase inhibitor or a prodrug thereof or a pharmaceutically acceptable salt of the same, and/or ezetimibe or a prodrug thereof or a pharmaceutically acceptable salt of the same,
and a package insert indicating that said pharmaceutical composition may be used or should be used for potentiating a blood cholesterol lowering action with a compound of the formula (1): 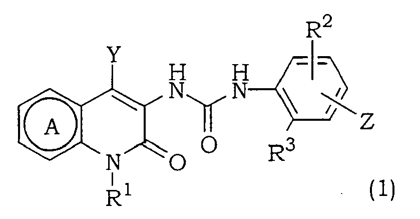 wherein Ring A is a substituted or unsubstituted pyridine ring;
Y is a substituted or unsubstituted alkyl group, a substituted or unsubstituted cycloalkyl group, or a substituted or unsubstituted aromatic group;
R¹ is a hydrogen atom, a substituted or unsubstituted alkyl group, a substituted or unsubstituted alkenyl group, a substituted or unsubstituted alkynyl group, or a substituted or unsubstituted cycloalkyl group;
R² is a hydrogen atom or a lower alkyl group;
R³ is a lower alkyl group;
Z is a group represented by either of the following formula 1) or 2):
1) ―D¹―Q
wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸― (R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or
2) ―D²―M―E―W
wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶― (R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond, or a prodrug thereof or a pharmaceutically acceptable salt of the same.

12. A commercial package which comprises a combination of
(A) a pharmaceutical composition comprising a compound of the formula (1): 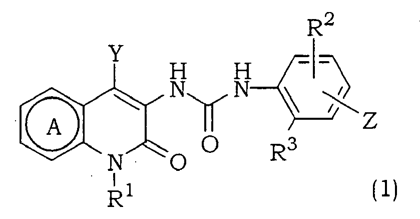 wherein Ring A is a substituted or unsubstituted pyridine ring;
Y is a substituted or unsubstituted alkyl group, a substituted or unsubstituted cycloalkyl group, or a substituted or unsubstituted aromatic group;
R¹ is a hydrogen atom, a substituted or unsubstituted alkyl group, a substituted or unsubstituted alkenyl group, a substituted or unsubstituted alkynyl group, or a substituted or unsubstituted cycloalkyl group;
R² is a hydrogen atom or a lower alkyl group;
R³ is a lower alkyl group;
Z is a group represented by either of the following formula 1) or 2):
1) ―D¹―Q
wherein D¹ is a direct bond or a divalent hydrocarbon group having 1 to 8 carbon atoms and optionally containing an unsaturated bond, Q is a hydroxy group, a carboxyl group, a substituted or unsubstituted heteroaryl group, or a group of the formula: ―NR⁴R⁵ (R⁴ and R⁵ are independently a hydrogen atom, a lower alkoxy-substituted or unsubstituted lower alkyl group, a cycloalkyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸―(R⁸ is a hydrogen atom, a substituted or unsubstituted lower alkyl group, a substituted or unsubstituted phenyl group, a substituted or unsubstituted benzyl group, or a lower alkoxycarbonyl group) or one oxygen atom in the cycle thereof), provided that when Q is a substituted or unsubstituted heteroaryl group, then D¹ is not a direct bond, or
2) ―D²―M―E―W
wherein D² is a direct bond or a divalent hydrocarbon group having 1 to 8 carbon atoms and optionally containing an unsaturated bond, M is an oxygen atom, a sulfur atom, a sulfinyl group or a sulfonyl group, or a group of the formula: ―NHC(=O)―, ―C(=O)NH― or ―NR⁶―(R⁶ is a hydrogen atom or a lower alkyl group), E is a direct bond or a divalent hydrocarbon group having 1 to 8 carbon atoms and optionally containing an unsaturated bond, W is a hydroxy group, a carboxyl group, a substituted or unsubstituted heteroaryl group, or a group of the formula: ―NR⁴R⁵ (R⁴ and R⁵ are as defined above), provided that when W is a hydroxy group, a carboxyl group or a group of the formula: ―NR⁴R⁵, then E is not a direct bond,
or a prodrug thereof or a pharmaceutically acceptable salt of the same; and
(B) 3-hydroxy-3-methylglutaryl coenzyme A reductase inhibitor or a prodrug thereof or a pharmaceutically acceptable salt of the same, and/or ezetimibe or a prodrug thereof or a pharmaceutically acceptable salt of the same;
and a package insert indicating that said combination may be used or should be used for lowering blood cholesterol.
